# EUROPEAN PATENT SPECIFICATION

(11) **EP 0 807 634 B1**
(45) Date of publication and mention of the grant of the patent: **09.04.2003**
(21) Application number: 97303062.0
(22) Date of filing: 06.05.1997
(51) Int. Cl.: C07F 1/08, C07C 405/00

(54) **Misoprostol, process for its preparation using an organometallic cuprate complex**
Misoprostol, Verfahren zur deren Herstellung mit Verwendung eines Organometall-Kupferkomplexes
Misoprostol, procédé pour le préparer utilisant un complexe cuivreux organométallique

(30) Priority: 17.05.1996 US 649402; 17.05.1996 CA 2176763
(43) Date of publication of application: 19.11.1997
(73) Proprietor: TORCAN CHEMICAL Ltd, Ontario, Canada L4G 1X9 (CA)
(72) Inventor: Lu, Yee Fung, Scarborough, Ontario, M1V 4X7 (CA); So, Raymond, Scarborough, Ontario M1W 3X8 (CA); To, Dan, Richmond Hill, Ontario, L4C 0E6 (CA); Antczak, Casimir, Aurora, Ontario L4G 5N5 (CA)
(74) Representative: Perry, Robert Edward

(56) References cited:
- US-A- 4 087 621
- US-A- 4 904 820
- WATRELOT, SANDRINE ET AL: "Stereoselective Synthesis of Allyltins from Vinyltins: A New Route to Enantioenriched.alpha.-Substituted (.gamma.-Alkoxyallyl)tins from Vinyltin Acetals" J. ORG. CHEM. (1994), 59(26), 7959-61 CODEN: JOCEAH;ISSN: 0022-3263, 1994, XP002075074

## Description

### FIELD OF THE INVENTION

This invention relates to organometallic synthetic processes and organometallic compounds useful therein. More specifically, it relates to methods of making organo-copper compounds which are useful in preparation of pharmaceutically active synthetic prostaglandin-type compounds.

### BACKGROUND OF THE INVENTION AND PRIOR ART

Canadian Patent 1,040,197 Pappo et. al. describes 16-oxygenated prostanoic acid derivatives and processes for their preparation. At least one of the compounds described in this patent, namely (11α,13E)-(±)-11,16-dihydroxy-16-methyl-9-oxoprost-13-en-1-oic acid, methyl ester, the generic name of which is misoprostol, has gained significant pharmaceutical and commercial acceptance as an inhibitor of gastric acid secretion. It has the following structural chemical formula:

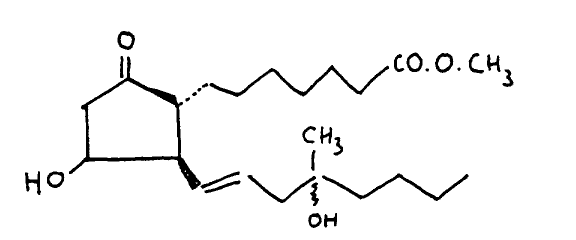

The synthetic method for preparing misoprostol and similar compounds disclosed in the aforementioned Pappo et.al. patent involves the preparation of a lower order lithium cuprate having unsaturated organic radicals associated with the copper ion, and the reaction of this lower order lithium cuprate with an appropriately chosen cyclopentenone, which in the case of misoprostol synthesis is methyl 7-(3RS)-tetrahydropyran-2-yloxy-5-oxocyclopent-1-ene)heptanoate. The lower order lithium cuprate used in this process, which in the case of misoprostol synthesis can be represented by the formula: 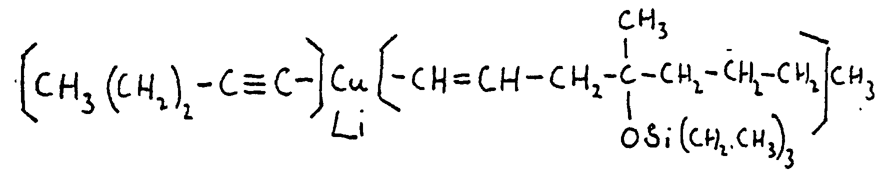 is made by a relatively complicated, multi-step process. The process involves reaction of an acetylenic alcohol with a trialkylsilyl halide to obtain the corresponding trialkylsilyl ether, addition of diisobutyl aluminum hydride across the acetylenic bond to produce the corresponding alkenyl aluminum derivative, reaction of this with iodine to obtain the silyl-protected 1-alkenyl iodide, and contact of this 1-alkenyl iodide with a lithium alkyl to form 1-alkenyl lithium which reacts with a cuprous acetylide to form the required lithium cuprate reagent.

Canadian patent 1,311,490 Campbell et.al. describes an improved and simplified process for preparing prostaglandins such as misoprostol, using higher order cuprates which will undergo conjugate addition to cyclopentenones. The higher order cuprates are prepared from vinyl stannane compounds, which are much easier to prepare than the vinyl iodides used in the Pappo et. al. patent process. These higher order cuprates are formed by reacting a higher order cuprate complex of formula: 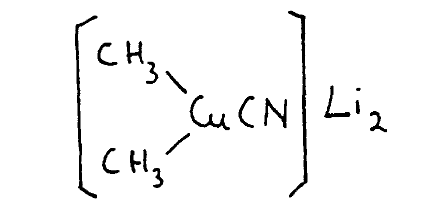 with a stannane of general formula Rₜ.Sn.(R₂)₃. The group Rₜ from the stannane, which is a carbanion and is normally an unsaturated group corresponding to the side chain required in the final prostaglandin compound, exchanges with one of the methyl groups on the cuprate complex, presumably to form a mixed higher order cuprate of the formula : 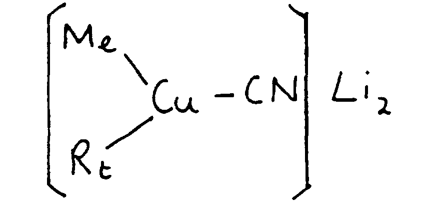 Alternative ligands to cyanide suggested in the Campbell et.al. patent are thiocyanate -SCN, sulfonyl-trifluoromethyl and thiophenyl, although only cyanide and thiocyanate are specifically exemplified.

It appears to be essential that the complex with vinyl group-containing ligands for reaction with enones such as cyclopentenones in prostaglandin synthesis be a copper complex. The straightforward reaction sequence to produce such a complex, where stannanes carrying the required vinyl group for addition onto the cyclopentenone nucleus are to be used, would be reaction of the appropriate vinyl stannane compound with an alkyl lithium such as n-butyl lithium to form a lithium vinyl species. This lithium vinyl compound could then be reacted with an organometallic copper compound, to exchange the vinyl group from the lithium compound with an organic radical from the copper compound, and hence produce the cuprate complex ready for reacting with the enone. However, the reaction of the alkyl lithium with the vinyl stannane will only take place at extremely low temperatures. The process disclosed in the Campbell et.al. patent overcomes this problem by first reacting the alkyl lithium with cuprous cyanide to obtain a first higher order complex, which is capable of reacting with the vinyl stannane at reasonable temperatures, to form a higher order cuprate capable of appropriate reaction with an enone.

The presence of ligands such as cyanide, with an empty n electronic orbital, on the cuprate complex has heretofore been believed to be necessary for the formation of higher order cuprate complexes with lithium alkyls; see "Organometallics in Synthesis: A Manual", Chapter 4, page 283-382; B.H. Lipshutz, Edited by M Schlosser, John Wiley & Sons (1994). According to prior art teachings, copper halides such as iodides and bromides do not form higher order cuprates, making necessary the use of copper cyanide and the like as starting materials; see Lipshutz, *supra,* and Synthesis 325 (1987). This is unfortunate and undesirable, because of the highly toxic nature of copper cyanide. This is hazardous to handle, and severely complicates waste disposal problems of wash waters from a process which involves its use.

Watrelot *et al*, J. Org. Chem (1994), 59(26), 7959-61, show the reaction of methyl lithium with cuprous iodide to produce a compound subsequently reacted with a vinyltin acetal to produce (δ-ethoxyallyl)tins. The molar ratio of Li:Cu is 2, and the resulting tin complex is not shown to be reactive with cyclopentenones.

### SUMMARY OF THE INVENTION

According to the present invention, it has been discovered that organometallic cuprate complexes capable of reacting with enones to effect addition of an organic radical from the cuprate onto the enone, can be prepared by reaction of an alkyl lithium compound with a cuprous halide and reaction of the resulting complex with a vinyl stannane, provided that an excess amount of alkyl lithium is used initially and is present during the reaction to form the organometallic cuprate complex. Under such conditions, the vinyl group from the stannane successfully transfers to the cuprate, to form a complex which readily reacts with an enone e.g. a cyclopentenone to form a prostaglandin carrying the vinyl group originally in the stannane compound, as a substituent.

The process of the present invention thus not only adopts a new chemical approach, but also permits the use of simple and readily available starting materials, e.g. methyl lithium and a cuprous halide, and avoids the need for the highly toxic cuprous cyanide or the like. The reactions proceed smoothly at reasonable temperatures, to give good yields of intermediate and final products. Cuprous halides used in the process of the present invention copper (I) iodide, copper (I) bromide, copper chloride (I) and copper (I) fluoride, with the fluoride being the least preferred and the iodide and bromide being the most preferred.

The first stage in the process according to the invention is the reaction of the alkyl lithium (e.g. methyl lithium) with the cuprous halide. This requires a stoichiometry of two equivalents of methyl lithium and one equivalent of cuprous halide, to produce the lower order cuprate. The process of the invention, however, uses more than two equivalents of alkyl lithium, to provide a small excess of alkyl lithium in the reaction but not such a large excess as to promote excessive side reactions. However, if exactly two equivalents, or less than two equivalents, of alkyl lithium are used, little or no reaction is observed.

Thus according to one aspect of the present invention, there is provided a process for preparing an organometallic cuprate complex carrying an organic unsaturated ligand, said cuprate complex being capable of reaction with an enone to effect addition of the organic unsaturated ligand from the cuprate complex onto the enone, which comprises reacting at least 2.05 equivalents of lower alkyl lithium with one equivalent of a cuprous halide, and reacting the product thereof with a vinyl stannane compound of the general formula: 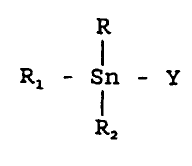 in which each of R, R₁, and R₂ is an independently selected lower alkyl radical and Y is an optionally substituted vinyl group.

### DESCRIPTION OF THE PREFERRED EMBODIMENTS

The process of the invention is best further described and illustrated with reference to the use of methyl lithium and cuprous iodide as the starting materials, the preferred embodiments thereof. However, it is not to be construed as limited thereto. It will be readily apparent that other lower alkyl (C₁ to C₆) lithium compounds and other cuprous halides as outlined above can also be used.

The chemical mechanism by which the process of the present invention proceeds is not currently fully understood. The presence of excess alkyl lithium e.g. methyl lithium is essential for the successful operation of the process according to the invention. If only the stoichiometric amount (2 equivalents thereof) is used, no complex able to react with an enone is formed.

The extent of excess of the methyl lithium needs to be only small. The reaction proceeds satisfactorily if only a very small, catalytic amount of methyl lithium is present, over and above the stoichiometric two equivalents, i.e. at least 2.05 equivalents of methyl lithium per equivalent of cuprous iodide.. The upper limit of methyl lithium amount is dictated primarily by the need to minimize side reactions. It is preferred for practical reasons, for example, to continue with the reaction of the cuprate complex with the enone to prepare the prostaglandin, without removing from the reaction mixture the by-products and unreacted starting materials from the synthesis of the cuprate complex. If there is too much residual methyl lithium at this stage, 1,2-addition to the ketone (cyclopentenone) may occur, to produce unwanted by-products and reduce overall yield. Preferably, therefore, the amount of methyl lithium does not exceed about 4 equivalents per equivalent of cuprous halide, the most preferred range being from about 2.1 : 1 to about 2.25 : 1, and the optimum being about 2.2 equivalents.

The group Y, initially part of the stannane reactant, can be substantially any vinyl group-containing radical which is desired to be substituted onto a cyclopentenone nucleus to form a pharmaceutically active prostaglandin compound. This group Y in general corresponds to the formula: 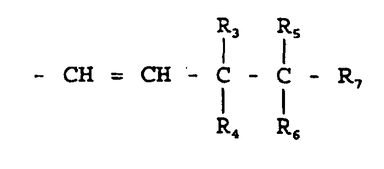 in which R₃, R₄, R₅, and R₆ are independently selected from hydrogen, C₁ to C₆ alkyl, C₁ to C₆ alkoxy, vinyl, hydroxy and protected hydroxy, and R₇ is C₂ to C₄ straight chain alkyl optionally interrupted by an ether linkage, or phenoxy. Preferred groups Y are those in which R₇ represents n-butyl and R₅ represents protected hydroxyl. Substantially any of the chemical groups conventionally used in organic synthetic chemistry to protect hydroxyl groups from chemical reaction, as set out in the standard literature, can be used as the hydroxyl protectant in the process of the present invention. Most preferred groups Y are those in which R₇ represents n-butyl, R₃ and R₄ represent hydrogen, R₆ represents methyl and R₅ represents protected hydroxyl, e.g. oxytrimethylsilyl protected hydroxyl.

The reaction of the excess methyl lithium with cuprous iodide suitably takes place in solution in an organic solvent, under strictly anhydrous conditions, since alkyl lithium compounds are extremely sensitive to water. The time of the reaction is suitably from about 5 minutes to two hours. The temperature of the reaction is suitably in the range -30°C - 10°C. Suitable solvents include tetrahydrofuran, dimethoxyethane, diethoxymethane, diethyl ether, diisopropyl ether, t-butylmethyl ether and the like.

The vinyl stannane compound, e.g. with R₁, R₂ and R being butyl, which is prepared separately, is added to the reaction mixture from the previous step. The conclusion of this reaction is signified by the presence of methyltributyl tin and the disappearance of the vinyl stannane compound and usually takes from about 5 minutes to about two hours. The order of addition of methyllithium or vinyl stannane compound is in general not critical and can be reversed. Methods of preparation of the appropriate vinyl stannane compounds are known, and form no part of the invention herein. In the case of misoprostol preparation using the process of the present invention, the vinyl stannane compound may have the formula:

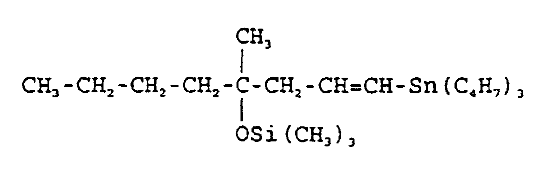

The vinyl stannane is added to the reaction mixture resulting from the reaction of the excess methyl lithium with cuprous iodide or bromide, without separating or isolating the reaction products and unreacted starting materials therefrom. The relative amounts of vinyl stannane and other reactants is relatively unimportant at this stage of the process, but is preferable about equimolar with the starting amount of cuprous iodide or bromide. The same organic solvent and generally the same reaction conditions as in the stage of reaction of the methyl lithium with the cuprous iodide or bromide can be used in this stage also. After the conclusion of the reaction of the vinyl stannane with the products of the earlier reaction, a process which normally takes about 5 minutes to two hours and the conclusion of which is indicated by the presence of methyltributyl tin and the disappearance of the vinyl stannane compound, the substituted cyclopentenone for formation of the prostaglandin can be added directly to the resulting reaction mixture, without the need to separate the resulting reaction products from the unreacted starting materials and by-products.

The preparation of the substituted cyclopentenone is also known procedure, and constitutes no part of the present invention. In the case of misoprostol preparation using the process of the present invention, the cyclopentenone may have the general formula, where Z is hydrogen or an appropriate protecting group:

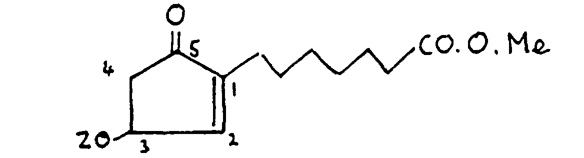

For preparation of other prostaglandin compounds by the process of the present invention (e.g. arbaprostil, gemeprost, trimoprostil, rioprostil, enprostil, enisoprost and viprostol), the substituent at position-1 of the cyclopentenone ring will differ from that for preparation of misoprostol, e.g. in the presence of ethylenic unsaturation in the chain and/or in the identity of the end group. Such differences do not alter the course of the reaction of the cuprate complex made according to the invention, to any significant extent. In general, the group at the 1-position of the cyclopentenone can be represented as R₈X, where R₈ represents a C₆ - C₇ straight-chain, saturated or unsaturated aliphatic hydrocarbon group, and X represents a carboxyl group, a lower alkyl esterified carboxyl group, a hydroxy group or a carbonyl-hydroxymethyl group. Similarly, other protectants may be used for the hydroxyl group at position-3.

The coupling reaction of the cuprate complex with the protected enone, which is in effect an addition reaction across the cyclopentene ring double bond, is effected by simply adding the protected enone to the reaction mixture in which the cuprate complex has been formed and is contained. The temperature of the reaction is suitably in the range from 0°C to -80°C, preferably -50°C to -80°C.

The crude, protected prostaglandin obtained can be deprotected and purified by standard methods to provide misoprostol.

The invention is further described, for illustrative purposes, with reference to the following specific examples.

### DESCRIPTION OF THE SPECIFIC, MOST PREFERRED EMBODIMENTS

### EXAMPLE 1

To a 1000 ml dried flask under a nitrogen atmosphere was added 74.6g of (E)-trimethyl[[1-methyl-1-[3-(tributylstannyl)-2-propenyl]pentyl]oxy]silane, 125 ml anhydrous THF and 24.2 g of copper (I) iodide. The mixture was stirred at room temperature for 30 minutes and then it was cooled to -25°C to -30°C. 98.8 ml of methyllithium (2.86 M) in DEM was added dropwise and the resultant solution was stirred at -15°C for 2 hours. Then the reaction mixture was cooled to -78°C to -80°C and 25g of methyl 5-oxo-3-[(triethylsilyl)oxy]-1-cyclopentene-1-heptanoate in 100 ml of THF was added rapidly. After stirring the mixture for 5 minutes at -78°C, it was quenched into a mixture of 750 ml of aqueous ammonium chloride solution and 200 ml of ammonium hydroxide. The resulting mixture was warmed to room temperature and stirred until a deep blue aqueous layer was obtained. Ethyl acetate (2x250 ml) was used for extraction. Then the combined organic layers were washed with brine (2x150 ml) and subsequently dried over magnesium sulfate. After a filtration and concentration under reduced pressure, an oil (105 g) was obtained. This oil containing the protected prostaglandin was subjected to acidic deprotection (cat.PPTS, acetone and water) and purification (chromatography on silica gel) to provide 15.8 g (60%) of misoprostol. This product was identical (¹H NMR, ¹³C NMR and IR) to a standard sample of misoprostol.

### EXAMPLE 2

To a 300 ml dried flask under a nitrogen atmosphere was added 4.45g of copper (I) iodide and 60 ml of anhydrous THF. The mixture was cooled to 0°C. 35 ml of 1.4M methyllithium in diethyl ether was added dropwise and the resultant solution was stirred at 0°C for 30 minutes. 13.7g of (*E*)-trimethyl[[1-methyl-1-[3-(tributylstannyl)-2-propenyl]pentyl]oxy]silanein 5 ml of THF was added and then the mixture was stirred at 0°C for 30 minutes. Then an additional 1.5 ml of 1.4M methyllithium in diethyl ether was added and the mixture was stirred at 0°C for another 30 minutes. The reaction mixture was cooled to -78°C and 10 g of methyl 5-oxo-3-[(triethylsilyl)oxy]-1-cyclopentene-1-heptanoate in 10 ml of THF was added rapidly. After stirring the mixture for 5 minutes at -78°C, it was quenched into 210 ml of basic aqueous ammonium chloride solution. The resulting mixture was warmed to room temperature and stirred until a deep blue aqueous layer was obtained. Ethyl acetate (2x200 ml) was used for extraction. Then the combined organic layers were washed with water (10 ml), then with brine (25 ml) and subsequently dried over magnesium sulfate. After a filtration and concentration under reduced pressure, an oil (21 g) was obtained. This oil containing the protected prostaglandin was subjected to acidic deprotection (cat.PPTS, acetone and water) and purification (chromatography on silica gel) to provide 4.2 g (40%) misoprostol.

## Claims

1. A process for preparing an organometallic cuprate complex carrying an organic unsaturated ligand, said cuprate complex being capable of reaction with an enone to effect addition of the organic unsaturated ligand from the cuprate complex onto the enone, which comprises reacting at least 2.05 equivalents of C₁-C₆ alkyl lithium with one equivalent of a cuprous halide, and reacting the product thereof with a vinyl stannane compound of the general formula: 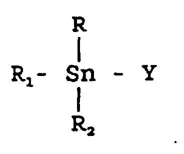 in which each of R, R₁, and R₂ is an independently selected lower alkyl radical and Y is an optionally substituted vinyl group.

2. The process of claim 1 wherein from 2.05 to 4 equivalents of alkyl lithium are reacted with 1 equivalent of cuprous halide.

3. The process of claim 1 wherein from 2.1 to 2.25 equivalents of alkyl lithium are reacted with 1 equivalent of cuprous halide.

4. The process of any preceding claim wherein the alkyl lithium compound is a C₁ - C₆ straight chain alkyl lithium, and the cuprous halide is copper (I) iodide or copper(I) bromide.

5. The process of any preceding claim wherein the alkyl lithium is methyl lithium.

6. The process of any preceding claim wherein group Y in the vinyl stannane compound corresponds to the formula: 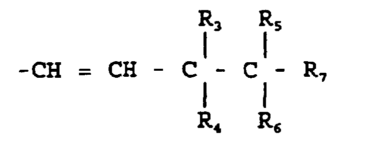 in which R₃, R₄, R₅, and R₆ are independently selected from hydrogen, C₁ to C₆ alkyl, C₁ to C₆ alkoxy, vinyl, hydroxy and protected hydroxy, and R₇ is C₂ to C₄ straight chain alkyl optionally interrupted by an ether linkage, or phenoxy.

7. The process of claim 6 in which R₇ represents n-butyl and R₅ represents protected hydroxyl.

8. The process of claim 6 or claim 7 including the further, subsequent step of reacting the organometallic cuprate complex so formed with a 1-substituted, 3-substituted, cyclopent-1-en-5-one of the general formula: 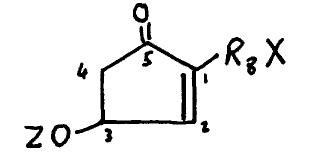 in which R₈ represents a C₆ - C₇ straight-chain, saturated or unsaturated aliphatic hydrocarbon group, and X represents a carboxyl group, a C₁-C₆ alkyl esterified carboxyl group, a hydroxy group or a carbonyl-hydroxymethyl group;
and Z represents hydrogen or a hydroxyl-protectant group; so as to effect 1,4-addition to the cyclopentenone and produce a synthetic prostaglandin with group Y at the 2-position of the cyclopentan-5-one nucleus.

9. The process of claim 8 wherein the substituted cyclopentenone compound is added to the reaction mixture resulting from the previous reaction step.

10. The process of claim 8 or claim 9 wherein the vinyl stannane reactant compound is (*E*)-trimethyl[[1-methyl-1-[3-(tributylstannyl)-2-propenyl]pentyl]oxy]silane, and the cyclopentenone compound is methyl 5-oxo-3-[(triethylsilyl) oxy]-1-cyclopentene-1-heptanoate, so as to prepare misoprostol as the final compound.

## Patentansprüche

1. Verfahren zur Herstellung eines organometallischen Cuprat-Komplexes, der einen organischen ungesättigten Liganden trägt, wobei der Cuprat-Komplex in der Lage ist, mit einem Enon zu reagieren, um die Addition des organischen ungesättigten Liganden vom Cuprat-Komplex an das Enon zu bewirken, welches umfasst das Umsetzen von mindestens 2,05 Äquivalenten C₁-C₆-Alkyllithium mit 1 Äquivalent eines Kupfer(I)-halogenids und das Umsetzen des Produkts davon mit einer Vinylstannan-Verbindung der allgemeinen Formel: 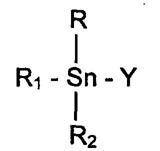 in welcher R, R₁ und R₂ jeweils ein unabhängig ausgewählter Niederalkylrest sind und Y eine gegebenenfalls substituierte Vinylgruppe ist.

2. Verfahren nach Anspruch 1, worin 2,05 bis 4 Äquivalente Alkyllithium mit 1 Äquivalent Kupfer(l)-halogenid umgesetzt werden.

3. Verfahren nach Anspruch 1, worin 2,1 bis 2,25 Äquivalente Alkyllithium mit 1 Äquivalent Kupfer(I)-halogenid umgesetzt werden.

4. Verfahren nach irgendeinem vorhergehenden Anspruch, worin die Alkyllithium-Verbindung ein geradkettiges C₁-C₆-Alkyllithium ist und das Kupfer(I)-halogenid Kupfer(I)-iodid oder Kupfer(I)-bromid ist.

5. Verfahren nach irgendeinem vorhergehenden Anspruch, worin das Alkyllithium Methyllithium ist.

6. Verfahren nach irgendeinem vorhergehenden Anspruch; worin die Gruppe Y in der Vinylstannan-Verbindung der Formel: 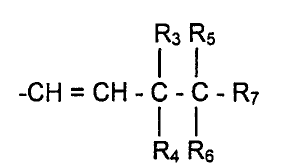 entspricht, in welcher R₃, R₄, R₅ und R₆ unabhängig ausgewählt sind aus Wasserstoff, C₁-C₆-Alkyl, C₁-C₆-Alkoxy, Vinyl, Hydroxy und geschütztem Hydroxy und R₇ geradkettiges C₂-C₄-Alkyl, gegebenenfalls unterbrochen von einer Etherverknüpfung, oder Phenoxy ist.

7. Verfahren nach Anspruch 6, in welchem R₇ n-Butyl ist und R₅ geschütztes Hydroxyl ist.

8. Verfahren nach Anspruch 6 oder Anspruch 7, das den weiteren folgenden Schritt der Umsetzung des so gebildeten organometallischen Cuprat-Komplexes mit einem 1-substituierten, 3-substituierten Cyclopent-1-en-5-on der allgemeinen Formel: 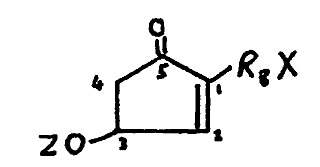 in welcher R₈ eine geradkettige, gesättigte oder ungesättigte, aliphatische C₆-C₇-Kohlenwasserstoffgruppe darstellt und X eine Carboxylgruppe, eine C₁-C₆-Alkyl-veresterte Carboxylgruppe, eine Hydroxygruppe oder eine Carbonylhydroxymethylgruppe ist und Z Wasserstoff oder eine Hydroxyschutzgruppe ist, beinhaltet, um die 1,4-Addition an das Cyclopentenon zu bewirken und ein synthetisches Prostaglandin mit Gruppe Y in 2-Position des Cyclopentan-5-on-Kerns herzustellen.

9. Verfahren nach Anspruch 8, worin die substituierte Cyclopentenon-Verbindung zu der Reaktionsmischung zugegeben wird, die sich aus dem vorherigen Reaktionsschritt ergibt.

10. Verfahren nach Anspruch 8 oder Anspruch 9, worin die Vinylstannan-Reaktantverbindung (E)-Trimethyl[[1-methyl-1-[3-(tributylstannyl)-2-propenyl]-pentyl]oxy]silan ist and die Cyclopentenon-Verbindung Methyl-5-oxo-3-[(triethylsilyl)oxy]-1-cyclopenten-1-heptanoat ist, um Misoprostol als Endverbindung herzustellen.

## Revendications

1. Procédé pour préparer un complexe de cuprate organométallique portant un ligand insaturé, organique, ledit complexe de cuprate étant capable de réagir avec une énone pour effectuer l'addition sur l'énone, du ligand insaturé, organique, du complexe de cuprate, qui consiste à faire réagir au moins 2,05 équivalents d'alkyllithium en C₁-C₆ avec un équivalent d'halogénure cuivreux, et à faire réagir le produit de ceci avec un composé de stannane de vinyle répondant à la formule générale: 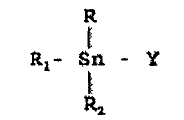 dans laquelle chacun parmi R, R₁ et R₂ est un radical alkyle inférieur choisi indépendamment, et Y est un groupe vinyle éventuellement substitué.

2. Procédé selon la revendication 1, dans lequel de 2,05 à 4 équivalents d'alkyllithium sont mis à réagir avec 1 équivalent d'halogénure cuivreux.

3. Procédé selon la revendication 1, dans lequel de 2,1 à 2,25 équivalents d'alkyllithium sont mis à réagir avec 1 équivalent d'halogénure cuivreux.

4. Procédé selon l'une quelconque des revendications précédentes, dans lequel le composé d'alkyllithium est un alkyllithium à chaîne droite, en C₁-C₆, et l' halogénure cuivreux est l'iodure de cuivre (I) ou le bromure de cuivre (I).

5. Procédé selon l'une quelconque des revendications précédentes, dans lequel l'alkyllithium est du méthyllithium.

6. Procédé selon l'une quelconque des revendications précédentes, dans lequel le groupe Y dans le composé de stannane de vinyle correspond à la formule: 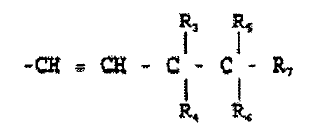 dans laquelle R₃, R₄, R₅ et R₆ sont indépendamment choisis parmi l'hydrog un alkyle en C₁ à C₆, un alkoxy en C₁ à C₆, un vinyle, un hydroxy et un hydroxy protégé, et R₇ est un alkyle à chaîne droite en C₂ à C₄, éventuellement interrompu par une liaison éther, ou un phénoxy.

7. Procédé selon la revendication 6, dans lequel R₇ représente le n-butyle, et R₈ représente un hydroxyle protégé.

8. Procédé selon la revendication 6 ou la revendication 7, comprenant l'étape subséquente, supplémentaire, consistant à faire réagir le complexe de cuprate organométallique ainsi formé avec une cyclopent-1-én-5-one 1-substituée, 3-substituée répondant à la formule générale: 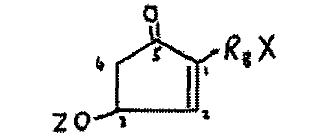 dans laquelle R₈ représente un groupe hydrocarbure aliphatique saturé ou insaturé, à chaîne droite, en C₆-C₇, et X représente un groupe carboxyle, un groupe carboxyle estérifié par un alkyle en C₁-C₆, un groupe hydroxy ou un groupe carbonyl-hydroxyméthyle;
et Z représente l'hydrogène ou un groupe protecteur d'hydroxyle; de manière à effectuer l'addition en 1,4, à la cyclopenténone, et à produire une prostaglandine synthétique avec un groupe Y au niveau de la position 2 du noyau cyclopentan-5-one.

9. Procédé selon la revendication 8, dans lequel le composé de cyclopenténone substitué est ajouté au mélange réactionnel résultant de l'étape de réaction précédente.

10. Procédé selon la revendication 8 ou la revendication 9, dans lequel le composé réactif au stannane de vinyle est le (E)-triméthyl[[1-méthyl-1-[3-(tributylstannyl)-2-propényl]pentyl]oxy]silane, et le composé de cyclopenténone est le méthyl 5-oxo-3-[(triéthylsilyl)oxy]-1-cyclopentène-1-heptanoate, de façon à préparer du misoprostol comme produit final.
